# EUROPEAN PATENT SPECIFICATION

(11) **EP 0 346 594 B1**
(45) Date of publication and mention of the grant of the patent: **31.05.1995**
(21) Application number: 89107037.7
(22) Date of filing: 19.04.1989
(51) Int. Cl.: C12Q 1/68, C12N 15/00

(54) **Recombinant RNA probes and methods of usage**
Rekombinante RNS-Proben und Verfahren zur Anwendung
Sondes d'ARN recombinant et méthodes pour leur utilisation

(30) Priority: 20.04.1988 US 183838
(43) Date of publication of application: 20.12.1989
(73) Proprietor: THE TRUSTEES OF COLUMBIA UNIVERSITY IN THE CITY OF NEW YORK, New York, New York 10027 (US)
(72) Inventor: Kramer, Fred R., New York, N.Y. 10463 (US); Lizardi, Paul M., Colonia Rancho Cortes CuernaVaca 62120 (MX)
(74) Representative: Henkel, Feiler, Hänzel & Partner

(56) References cited:
- WO-A-87/06270
- US-A- 4 786 600
- JOURNAL OF MOLECULAR BIOLOGY, vol. 171, 1983, Academic Press Inc., London (GB); E.A. MIELE et al., pp. 281-295#
- BIOCHEMICAL & BIOPHYSICAL RESEARCH COMMUNICATIONS, vol. 64, no. 2, 1975, Academic Press Inc., New York, NY (US); M. OBINATA et al., pp. 640-647#
- NUCLEIC ACIDS RESEARCH, vol. 14, no. 14, 1986, IRL Press Ltd., Oxford (GB); B.C.F. CHU et al., pp. 5591-5603#
- COLD SPRING HARBOUR SYMPOSIA ON QUANTITATIVE BIOLOGY, vol. 33, 1968; pp. 101-124#
- PROCEEDINGS OF THE NATL. ACADEMY OF SCIENCES USA, vol. 69, no. 10, 1972; pp. 3038-3042#
- SCIENCE, vol. 180, 1973; pp. 916-927#
- JOURNAL OF MOLECULAR BIOLOGY, vol. 117, no. 4, 1977; pp. 877-907#
- COLD SPRING HARBOUR SYMPOSIA ON QUANTITATIVE BIOLOGY, vol. LII, 1987; pp. 321-330#
- GENE, vol. 2, 1977; pp. 95-113#

## Description

The present invention concerns a replicatable and hybridizable recombinant single-stranded RNA probe molecule and the use of the same in a method for determining the presence or concentration of a nucleic acid sequence

### Background of the Invention

It is now a well established fact that all living organisms including infectious agents, e.g., viruses, contain DNA, or sometimes RNA, molecules which carry genetic information in the form of a nucleotide sequence code. While certain segments of this code are shared by many organisms, there are other segments which contain nucleotide sequences that are unique for a particular organism. These sequences are said to be species-specific and provide a convenient tag or footprint that can be utilized for identification of that organism. The technique of nucleic acid hybridization (1) has great potential for the rapid detection and typing of infectious agents. However, current hybridization assays have not yet attained the sensitivity and speed required for practical diagnostic use. It has recently been proposed that the sensitivity and speed of bioassays could be improved by linking a replicatable RNA to a hybridization probe (2). After hybridization, the replicatable RNA would be amplified by incubation with the RNA-directed RNA polymerase, Qβ replicase (3). The enormous number of RNA copies that would be synthesized would serve as a signal that hybridization had occurred. The synthesis of novel nucleic acid hybridization probes are reported in this invention that combine in a single RNA molecule the dual functions of probe and amplifiable reporter.

A distinguishing feature of RNA synthesis by Qβ replicase is that a small number of template strands can initiate the synthesis of a large number of product strands (4). Million-fold increases in the amount of RNA routinely occur in vitro (5) as a result of an autocatalytic reaction mechanism (6,7): single-stranded RNAs serve as templates for the synthesis of complementary single-stranded products; after the completion of product strand elongation, both the product and the template are released from the replication complex (8); and both strands are free to serve as templates in the next round of synthesis. Consequently, as long as there is an excess of replicase, the number of RNA strands increases exponentially. After the number of RNA strands equals the number of active replicase molecules, RNA synthesis continues linearly. There are a number of advantages to using the amplification of RNA by Qβ replicase as the basis of a signal-generating system: Qβ replicase is highly specific for its own template RNAs (9); as little as one molecule of template RNA can, in principle, initiate replication (10); and the amount of RNA synthesized (typically, 200 ng in 50 »l in 15 minutes) is so large that it can be measured with the aid of simple colorimetic techniques.

Two developments led to the current application: 1) the discovery that heterologous RNA segments can be inserted within the sequence of a small, naturally occurring template for Qβ replicase, MDV-1 RNA (11), without affecting its replicability (12); and 2) the construction of a plasmid that serves as a template for the synthesis of MDV-1 (+) RNA when the plasmid is incubated in vitro with bacteriophase T7 RNA polymerase (13).

This plasmid has been modified in the present invention by inserting a polylinker within the MDV-1 cDNA sequence, and then inserting synthetic hybridization probe sequences within the polylinker. The resulting plasmids served as templates for the synthesis of "recombinant RNAs," which consist of a probe sequence embedded within the sequence of MDV-1 (+) RNA. The probe sequences employed in this invention and which will be further described in the Experimental Details section to follow, are known to hybridize specifically to the repetitive DNA of Plasmodium falciparum (14-16), one of the protozoans that cause malaria. In this invention, it will be shown that these recombinant RNA molecules are bifunctional, in that they are able to hybridize specifically to complementary DNA targets and they are also able to serve as templates for exponential amplification by Qβ replicase.

In Kramer et al., U.S Patent No. 4,786,600, issued November 22, 1988, there are disclosed replicatable recombinant single-stranded RNA molecules comprising a recognition sequence for the binding of an RNA-directed RNA polymerase, a sequence for the initiation of product strand synthesis by the polymerase and a heterologous sequence of interest derived from a different RNA molecule inserted at a specific site in the internal region of the recombinant molecule. Kramer et al. does not teach or suggest that if the inserted sequence is a hybridization probe sequence, that the resulting molecules can be replicated after hybridization to produce multiple copies for detection.

In Chu et al., U.S. Application Serial No. 852,692, filed April 16, 1986, and WO-A-8 706 270 methods are disclosed for determining the presence of targets, i.e., analytes, by linking a replicatable RNA, which serves as a reporter group, to a probe, e.g., an oligonucleotide, an antibody or lectin. Chu et al. also disclose that an RNA-directed RNA polymerase can then be used to after hybridization has occurred to produce multiple copies of the replicatable RNA for detection. However, Chu et al. do not describe a method in which different recombinant-RNA "probes" sequences can be used simultaneously in the same assay. Example IX of WO-A-8 706 270 describes a recombinant RNA molecule which consists of MDV-1 RNA with the 30-mer oligoribonucleotide 5'-AGUCAGGCACCGUGUAUGAAAUCUAACAAU-3' inserted between the G at position 63 and the U at position 64 of the MDV-1 sequence.

### Summary of the Invention

The present invention concerns a replicatable and hybridizable recombinant single-stranded RNA probe molecule comprising: a recognition sequence for the binding of an RNA-directed RNA polymerase; a sequence required for the initiation of product strand synthesis by the polymerase; and a heterologous RNA sequence, complementary to a specific nucleic acid sequence of an infectious agent, or a heterologous RNA sequence other than the sequence 5'-AGUCAGGCACCGUGUAUGAAAUCUAACAAU-3', complementary to a specific gene sequence or portion thereof, either heterologous RNA sequence being, inserted at a specific site in the internal region of the recombinant molecule and complementary to an oligo- or polynucleotide of interest, such that the heterologous sequence of the probe molecule may have single-stranded conformation for hybridization with a target sequence and may form secondary structures for efficient replication.

The invention also provides a method for determining the presence or concentration of an oligo- or polynucleotide of interest in a sample, comprising the steps of: (a) forming a specific complex between the recombinant-RNA probe molecule of claim 1 and the oligo- or polynucleotide of interest, by incubating the sample with the recombinant-RNA probe molecules under suitable conditions and for a sufficient period of time to permit complementary nucleotide sequences to hybridize; (b) removing unhybridized recombinant-RNA probe molecules from the reaction mixture; (c) incubating the reaction mixture with an RNA-directed RNA polymerase capable of synthesizing additional copies of the recombinant-RNA probe molecules that are hybridized to the oligo- or polynucleotide of interest; and (d) detecting the recombinant-RNA probe molecules synthesized in step (c), thereby determining the presence or concentration of the oligo- or polynucleotide of interest.

The invention herein further provides a method for simultaneously determining the presence or concentration of several different oligo- or polynucleotides of interest in a sample, comprising the steps of: (a) forming specific complexes between a mixture of different types of recombinant-RNA probe molecules of claim 1, each type having a different inserted sequence, and the oligo- or polynucleotides of interest, by incubating the sample with the mixture of recombinant-RNA probe molecules under suitable conditions and for a sufficient period of time to permit complementary nucleotide sequences to hybridize; (b) removing unhybridized recombinant-RNA probe molecules from the reaction mixture; (c) incubating the reaction mixture with an RNA-directed RNA polymerase capable of synthesizing additional copies of the recombinant-RNA probe molecules which are hybridized to the oligo- or polynucleotides of interest; (d) separating the mixture of synthesized recombinant-RNAs by hybridizing them to an ordered array of polynucleotides bound to a membrane, where each of the polynucleotides is complementary to one type of synthesized recombinant-RNA; and (e) detecting the recombinant-RNA probe molecules produced in step (d), thereby determining the presence or concentration of each oligo- or polynucleotide of interest.

### Brief Description of the Figures

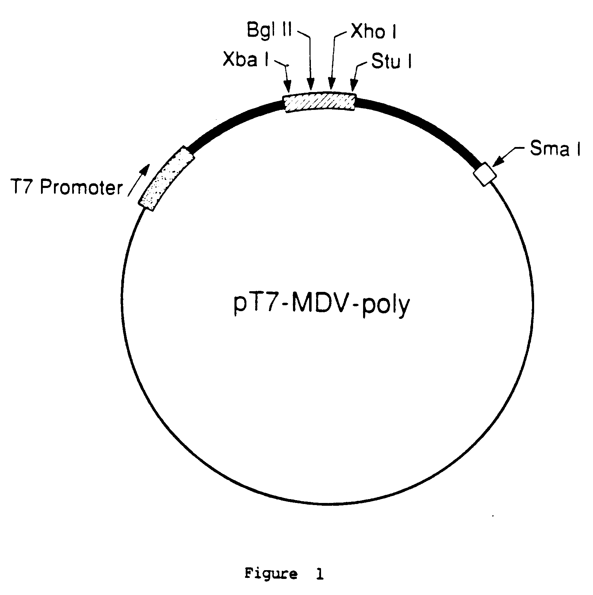

Figure 1. Structure of plasmid pT7-MDV-poly. The heavy black line represents MDV-1 cDNA. When this plasmid is cleaved with endonuclease Sma I and incubated in vitro with T7 RNA polymerase, the resulting transcripts are replicatable RNAs.

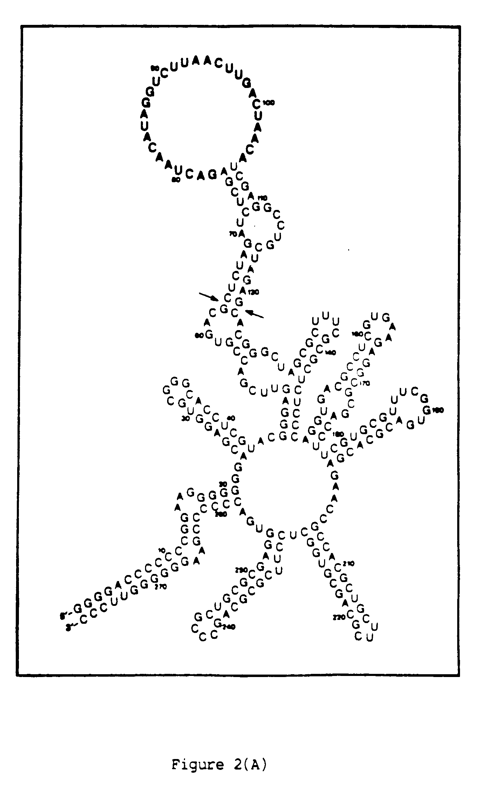

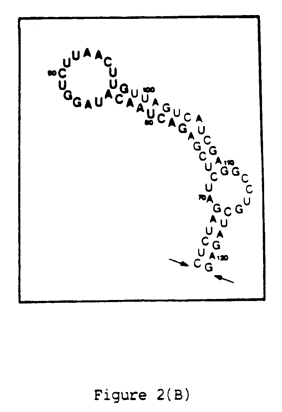

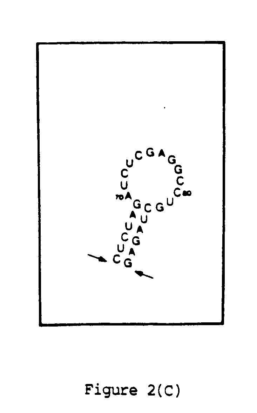

Figure 2. Nucleotide sequences of the recombinant transcripts folded into the secondary structures predicted by a computer program to be most stable (41). MDV-fal-un (+) RNA (A) contains a 58-nucleotide insert (shown between the arrows) in place of the 3-nucleotide segment, AGU, that occurs in natural MDV-1 (+) RNA (42). The secondary structures that the computer program predicts in the region of the recombinant outside the insert are identical to the secondary structures that were experimentally identified in MDV-1 RNA (36,37), suggesting that the probe sequence would have little effect on the topology of the MDV-1 domain. Bold letters indicate the nucleotides that are complementary to P. falciparum DNA. Figures 2(B) and 2(C) show the probable secondary structure of the inserts present in MDV-fal-st (+) RNA (B) and MDV-poly (+) RNA (C).

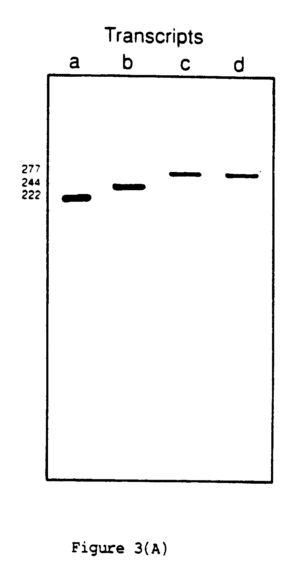

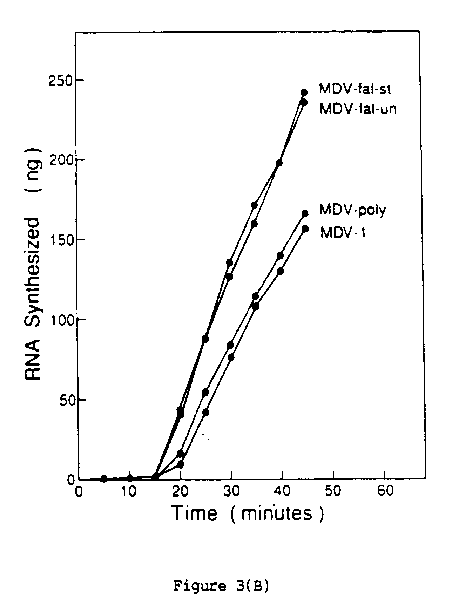

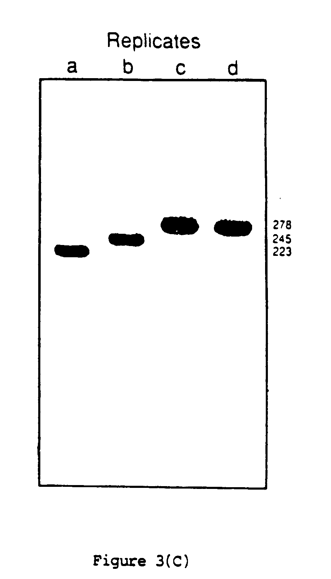

Figure 3. Replication of recombinant RNAs. (A) Polyacrylamide gel electrophoresis showed the relative mobility of different transcripts: MDV-1 (a), MDV-poly (b), MDV-fal-un (c), and MDV-fal-st (d). The numbers on the side of the panel indicate the length of each transcript (in nucleotides). (B) Qβ replicase reactions were initiated with 200 fg of each transcript. Kinetic analysis showed that approximately 100,000 copies of each transcript were synthesized in 20 minutes. (C) Electrophoretic analysis of the RNA present after 45 minutes showed that the products were replicates of the transcripts. The products were probably one nucleotide longer than the transcripts, since a nonessential, 3′-terminal adenosine is usually added during replication (21).

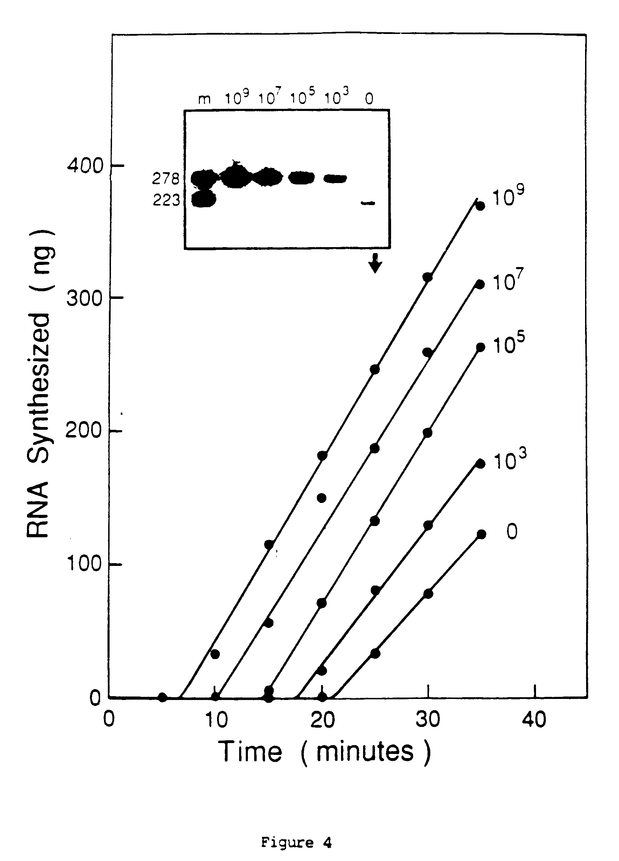

Figure 4. Effect of initial RNA concentration on the time-course of recombinant RNA synthesis. A series of 25-»l Qβ replicase reactions were initiated with the following quantities of MDV-fal-un RNA: 140 pg, 1.4 pg, 14 fg, 0.14 fg, and 0 fg, Which corresponds to 10⁹, 10⁷ 10⁵, 10³, and 0 molecules of added template. Samples of each reaction were taken every 5 minutes to determine the amount of RNA that had been synthesized. The results demonstrate that the time spent in the exponential phase of synthesis is increased when the initial number of template molecules is decreased (5). Each 100-fold reduction in the number of recombinant molecules used as template resulted in a 3.59 minute delay in achieving saturation (indicating that the RNA population doubled every 32.4 seconds). The electrophoretic mobility of the RNA present in each reaction after 25 minutes (see inset), compared with the mobility of recombinant RNA (278-nucleotides long) and MDV-1 RNA (223-nucleotides long) markers (m), confirmed that the product of reactions initiated with recombinant RNA was recombinant RNA.

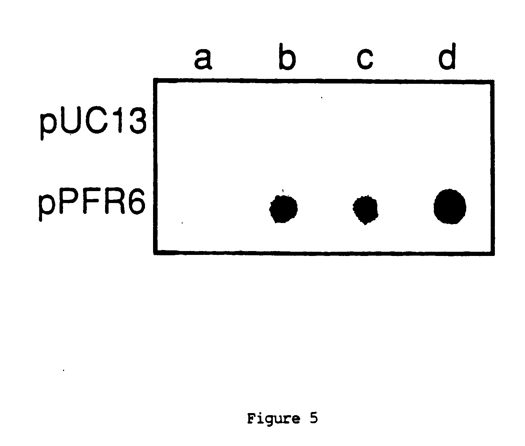

Figure 5. Hybridization of recombinant RNAs. 28 ng MDV-poly RNA (a), 75 ng MDV-fal-st RNA (b), and 75 ng MDV-fal-un RNA (c) where hybridized to dot-blots (34) containing either 0.5 »g pPFR6 DNA (a plasmid containing 45 copies of the P. falciparum target sequence) or 0.5 »g pUC13 DNA (the plasmid vector used to construct pPFR6). The recombinant RNA hybridized specifically to plasmids containing target sequences. When the amount of both the MDV-fal-un RNA and the DNA was doubled (d), a marked increase was seen in the amount of RNA that bound to the target DNA.

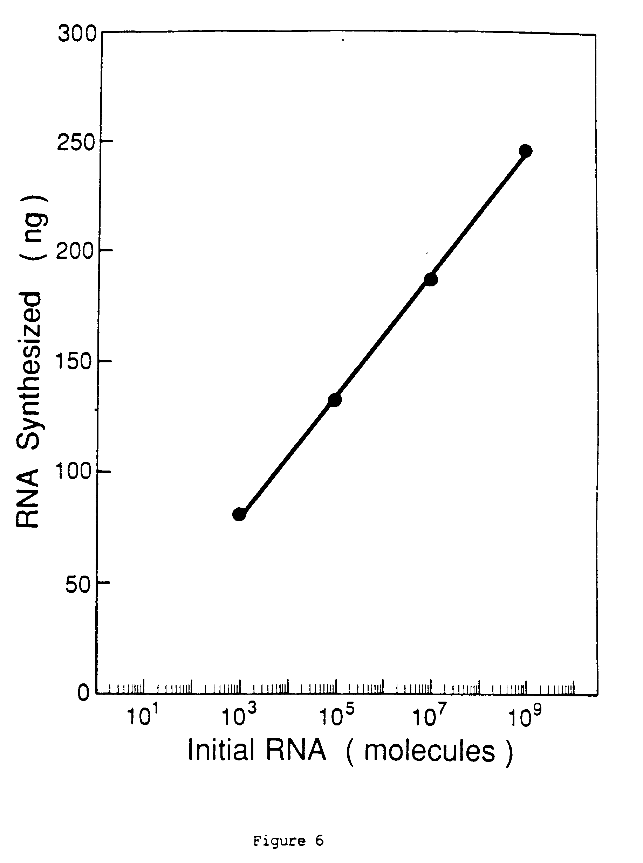

Figure 6. Linear relationship between the logarithm of the initial number of RNA molecules added to a Qβ replicase reaction and the amount of product RNA synthesized. The amount of RNA synthesized in 25 minutes in the reactions shown in Figure 4 was plotted against the logarithm of the number of recombinant molecules that had been added initially to each reaction. These results illustrate how bioassays that utilize replicatable probes would be interpreted: the amount of product RNA would be proportional to the logarithm of the number of probes bound to targets.

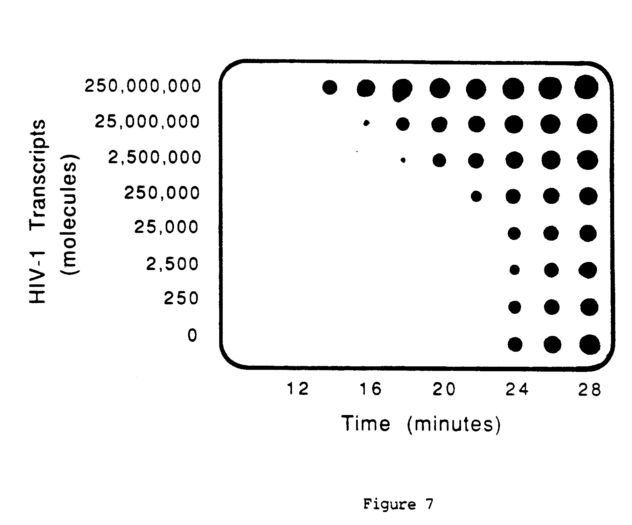

Figure 7. Autoradiogram of hybridization reactions showing amount of RNA present at each time point.

### Detailed Description of the Invention

This invention concerns a replicatable and hybridizable recombinant single-stranded RNA probe molecule comprising a recognition sequence for the binding of an RNA-directed RNA polymerase; a sequence required for the initiation of product strand synthesis by the polymerase; and a heterologous RNA sequence, complementary to a specific nucleic acid sequence of an infectious agent, or a heterologous RNA sequence other than the sequence 5'-AGUCAGGCACCGUGUAUGAAAUCUAACAAU-3', complementary to a specific gene sequence or portion thereof, either heterologous RNA sequence being, inserted at a specific site in the internal region of the recombinant molecule and complementary to an oligo- or polynucleotide of interest, such that the heterologous sequence of the probe molecule may have single-stranded conformation for hybridization with a target sequence and may form secondary structures for efficient replication.

In one embodiment, the recognition sequence for the binding of an RNA-directed RNA polymerase is in an internal region of the recombinant-RNA probe molecule.

In another aspect of this invention, the insertion site for the heterologous RNA sequence is not near any sequence required for the binding of the RNA polymerase or for the initiation of product strand synthesis. In a further aspect, such an insert has a minimal affect on the replicability of the molecule. In a still yet further aspect of this invention, the insert in the recombinant-RNA probe molecule has a minimal effect upon the secondary and tertiary structure of the molecule. In the practice of this invention, the specific insertion site of the recombinant-RNA probe molecule is at a specific nucleotide. In the practice of this invention the heterologous sequence of interest is inserted between nucleotides 63 and 64.

The sequence in the recombinant RNA probe molecules of the present invention which is required for the initiation of product strand synthesis is a cytidine-rich 3'-terminal sequence.

An RNA-directed RNA polymerase useful in the practice of this invention is Qβ replicase.

Useful in to the present invention is a recombinant-RNA probe molecule wherein the molecule is a variant RNA template for Qβ replicase or a mutant thereof. In a further aspect, such a variant RNA template is MDV-1 RNA or a mutant thereof. In one embodiment, the MDV-1 RNA is MDV-1 (+) RNA. In another, the MDV-1 RNA is MDV-1 (-) RNA.

Transcripts derived from a recombinant plasmid by incubation with a DNA-directed RNA polymerase are especially useful in the practice of the invention to provide recombinant-RNA probe molecules. In the practice of this invention, the recombinant-RNA probe molecule is a variant RNA template for Qβ replicase or a mutant thereof. In a preferred embodiment, the variant RNA template is MDV-1 RNA or a mutant thereof. In still other embodiments, the MDV-1 RNA is MDV-1 (+) RNA or MDV-1 (-) RNA.

One feature of the invention herein is to provide a recombinant-RNA probe molecule wherein the heterologous sequence is inserted between nucleotides 63 and 64.

An important feature is obtained by this invention where the inserted heterologous sequence of the recombinant-RNA probe molecule is complementary to a specific nucleic acid sequence of an infectious agent. Such an infectious agent may be a virus, a viroid or virusoid, a prokaryote, a bacterium, a eukaryote, or a parasitic protozoan, such as the parasitic protozoan that causes malaria.

In a further aspect, the inserted heterologous sequence of the recombinant-RNA probe molecule may be complementary to a specific gene sequence or portion thereof, or to an allele of the specific gene sequence or portion thereof.

The invention herein provides a method for determining the presence or concentration of an oligo- or polynucleotide of interest in a sample, comprising the steps of: (a) forming a specific complex between the recombinant-RNA probe molecules, as described above, and the oligo- or polynucleotide of interest, by incubating the sample with the recombinant-RNA probe molecules under suitable conditions and for a sufficient period of time to permit complementary nucleotide sequences to hybridize; (b) removing unhybridized recombinant-RNA probe molecules from the reaction mixture; (c) incubating the reaction mixture with an RNA-directed RNA polymerase capable of synthesizing additional copies of the recombinant-RNA probe molecules that are hybridized to the oligo- or polynucleotide of interest; and (d) detecting the recombinant-RNA probe molecules synthesized in step (c), thereby determining the presence or concentration of the oligo- or polynucleotide of interest.

Such a method as described above may be used to generate highly amplified signals in a nucleic acid hyybridization assay. In theory the method has the power to generate a signal from a single hybridized molecule, and therefore, could be utilized to devise ultra-high sensitivity DNA (or RNA) detection assay. The method is based on the use of Qβ RNA recombinant constructs and Qβ Replicase.

In such a method, the oligo- or polynucleotide in the sample may be bound to a solid support. In such cases, the solid support may be a nitrocellulose or nylon membrane.

In carrying out the above-described method of this invention and forming a specific complex between the recombinant-RNA probe molecules and the oligo- or polynucleotide of interest, i.e., step (a), the oligo-or polynucleotide of interest and the recombinant-RNA probe molecule may be in solution.

The unhybridized recombinant RNA molecules may be separated from those that are hybridized to the oligo- or polynucleotides of interest by employing techniques and skills which are well-known in the art. In the usual case, with the recombinant RNA molecule hybridized to the oligo- or polynucleotide of interest, which in turn is bound to a solid support, such separation is readily accomplished by simple washing which does not significantly disrupt the connection to the solid support. In addition, a technique known as the sandwich hybridization method may also be used to effect separation of hybridized from unhybridized recombinant-RNA molecules. Chromatographic and electrophorectic techniques may be used as well. In a further aspect of the method described hereinabove, the unhybridized recombinant-RNA probe molecules are removed from the reaction mixture in step (b) by separating hybridized recombinant-RNA probe molecules from the unhybridized probe molecules through the capture of the oligo- or polynucleotide onto a solid support.

In detecting the recombinant-RNA probe molecules which have been synthesized or replicated in step (c) above, methods well-known to those in this art may be employed. For example, detection can be by ultraviolet absorbance of replicated RNA, as, for example, by the method of contact photoprinting (54).

In one embodiment detecting is carried out by the incorporation of radioactively labelled ribonucleoside 5′-triphosphate precursors into the recombinant-RNA products. In another embodiment, detecting is carried out by the incorporation of chemically modified ribonucleoside 5′-triphosphate precursors into the recombinant-RNA products.

Biotin or iminobiotin can be incorporated into replicated RNA, which can then be detected by known techniques with an enzyme-avidin or enzyme-streptavidin adduct, which binds to the RNA-bound biotin and catalyzes production of a conviently detectable chromogen. See Matthews (55); Leary et al. (45). Incorporation of biotin or iminobiotin into replicated RNA can be accomplished by employing UTP that is biotinylated through a spacer to carbon-5 of the uracil moiety as a substrate for the replicate in the replication reaction. Such UTP's are known compounds. Further, it is known that such UTP's are substrates for Qβ replicase, and that RNAs which include uracils biotinylated through spacer groups joined to the carbon-5 position, due to use of such UTP's in their synthesis, are templates for Qβ replicase catalyzed replication.

RNA resulting from the replication process could also be biotinylated employing photobiotin acetate according to the procedure of Forster et al., (56), and then detected, with an avidin-enzyme adduct-chromogenic compound system, like replicated RNA's synthesized with biotinylated UTP in the replication reaction. Thus, in still another aspect, the chemically modified ribonucleoside 5′-triphosphate precursors may be biotinylated or the chemically modified ribonucleotide 5′triphosphate precursors may be fluorescent. A further feature of this method is obtained where detecting is carried out by the binding of RNA-specific chromogenic or fluorogenic dyes to the recombinant-RNA products. RNA resulting from the replication process can be made fluorescent by employing a T4 RNA ligase-catalyzed reaction to append nucleotides modified to be fluorescent to the 3′-end of replicative RNA. See Cosstick et al., Nucl. Acis Res. 12, 1791-1810 (57). The fluorescence of the resulting RNA can be employed to detect the RNA by any of the several standard techniques.

Among still other methods that can be used to detect replicated RNA are those wherein a reporter substance, that binds specifically with nucleic acid, is added to the system in which the replication has taken place, or to the medium, such as a positively charged support such as ECTEOLA® paper, on which replicated RNA has been isolated, and signal from the reporter substance measured. Such substances include: chromogenic dyes, such as "stains all" (Dahlberg et al., (58); methylene blue (Dingman and Peacock, (59), and silver stain (Sammons et al., 32); Igloie, (60)); fluorogenic compounds that bind to RNA -- for example, ethidium bromide (Sharp et al.,(61); Bailey and Davidson, (62); and fluorogenic compounds that bind specifically to RNAs that are templates for replication by Qβ replicase -- for example, a phycobiliprotein (Oi et al., (63); Stryer et al., U.S. Patent No. 4,520,110) conjugated to the viral subunit of Qβ replicase.

Detecting may be also carried out by physical methods, such as the absorption of ultraviolet light and the determination of mass by weighing.

In incubating the reaction mixture obtained in step (b), an RNA-directed RNA polymerase is employed. An example of such a polymerase useful in the practice of this invention is Qβ replicase.

This invention also provides recombinant-RNA probe molecules produced by the above-described method and in particular, recombinant-RNA probe molecules produced by a method where the reaction mixture is incubated with Qβ replicase to synthesize additional copies of the recombinant-RNA probe molecules that are hybridized to the oligo- or polynucleotide of interest.

Another aspect of the method provided by this invention is attained when the time of incubation in step (c) is sufficiently short so that the number of recombinant-RNA product strands does not exceed the number of polymerase molecules, with the result that the number of recombinant-RNA product molecules is linearly proportional to the number of recombinant-RNA probe molecules originally hybridized.

The method described herein provides another important feature where the time of incubation in step (c) is sufficiently long so that the number of recombinant-RNA product strands exceeds the number of polymerase molecules, with the result that the number of recombinant-RNA product molecules is proportional to the logarithm of the number of recombinant-RNA probe molecules originally hybridized.

This invention also provides a method for simultaneously determining the presence or concentration of several different oligo- or polynucleotides of interest in a sample, comprising the steps of: (a) forming specific complexes between a mixture of different types of recombinant-RNA probe molecules of claim 1, each type having a different inserted sequence, and the oligo-or polynucleotides of interest, by incubating the sample with the mixture of recombinant-RNA probe molecules under suitable conditions and for a sufficient period of time to permit complementary nucleotide sequences to hybridize; (b) removing unhybridized recombinant-RNA probe molecules from the reaction mixture; (c) incubating the reaction mixture with an RNA-directed RNA polymerase capable of synthesizing additional copies of the recombinant-RNA probe molecules that are hybridized to the oligo- or polynucleotides of interest; (d) separating the mixture of synthesized recombinant-RNAs by hybridizing them to an ordered array of polynucleotides bound to a membrane, where each of the polynucleotides is complementary to one type of synthesized recombinant-RNA; and (e) detecting the recombinant-RNA probe molecules produced in step (d), thereby determining the presence or concentration of each oligo- or polynucleotide of interest.

A good clinical assay requires speed, specificity, and sensitivity. For example, current assays for the identification of the pathogenic agents that cause acute bacterial meningitis lack sufficient speed and sensitivity. A number of different organisms are associated with meningitis, including, Haemophilus influenzae, Klebsiella pneumoniae, Neisseria meningitidis, Staphylococcus aureus, and Streptococcus pneumoniae. The patient is typically a young child. Effective treatment requires the identification of the etiologic agent in a sample of cerebrospinal fluid; and it is essential that antibiotic treatment begin as soon as possible. However, current laboratory techniques require at least 18 hours to identify the agent. Moreover, the sample volume is usually only 50 microliters and often contains less than 50 individual microorganisms, which is well below the limits at which reliable direct detection assays can currently be carried out (64). The clinical picture is further complicated by a marked increase in the abundance of bacteria that possess antibiotic resistance genes on plasmids (65,66).

A series of replicatable recombinant-RNA probes molecules can be prepared each of which is specific for a different organism that can cause meningitis. In addition, a series of recombinant-RNA probe molecules can be prepared, each of which is specific for different antibiotic resistance genes that could be present in the infectious agent.

Using such a series of recombinant-RNA probe molecules a mixture of, for example, 15 different replicatable recombinant-RNA probes are incubated with denatured DNA obtained from a sample of spinal fluid. Only a few types of recombinant-RNA probe molecules species will find targets (for example, one bacterial probe and three resistance gene probes). After removing the unbound probe molecules, Qβ replicase is used to amplify the remaining probes. After amplification, the mixture of product RNAs is placed in contact with a membrane containing numbered dot-blots, each of which contains denatured DNA complementary to one of the original probe sequences. In this manner, the mixture of product RNAs is sorted out for subsequent quantification. These assays permit the rapid and simultaneous diagnosis of both the pathogenic agent and its spectrum of antibiotic resistance.

In a similar manner a mixture of probes could be used simultaneously to detect the human immunodeficiency virus which causes acquired immune deficiency syndrome (AIDS) and the concentration of an entire panel of opportunistic agents that infect the immunosuppressed patient.

This invention is illustrated in the Experimental Details and Experimental Discussion sections which follow. These sections are set forth to aid in an understanding of the invention but are not intended to, and should not be constructed to, limit in any way the invention as set forth in the claims which follow thereafter.

### Experimental Details

### Material and Methods

### Enzymes

Restriction endonucleases, T4 polynucleotide kinase, and T7 RNA polymerase were purchased from New England Biolabs. Calf intestine alkaline phosphatase, T4 DNA ligase, the Klenow fragment of Escherichia coli DNA polymerase I, and bovine pancreatic deoxyribonuclease I were purchased from Boehringer Mannheim. Qβ replicase was isolated from bacteriophage Qβ-infected E. coli Q13 by the procedure of Eoyang and August (17), with the hydroxylapatite step omitted.

### Oligonucleotides

Single-stranded DNA fragments were prepared, using β-cyanoethyl phosphoramidite chemistry (18), in a Microsyn-1450A synthesizer (Systec). After deblocking and release from the resin, the oligonucleotides were isolated by preparative gel electrophoresis (19), eluted from the gel, filtered through nitrocellulose, and purified by chromatography (20) on SEP-PAK C18 cartridges (Waters Associates).

### Plasmid for Synthesizing MDV-1 (+) RNA by Transcription

pT7-MDV contains a promoter for T7 RNA polymerase directed towards a full-length cDNA prepared from MDV-1 RNA (13). This plasmid had been constructed so that transcription from the T7 promoter begins with the first nucleotide of MDV-1 (+) RNA. A Sma I restriction site had been introduced at the other end of the MDV-1 cDNA sequence so that when this plasmid is linearized by digestion with endonuclease Sma I and then incubated with T7 RNA polymerase, transcription terminates two nucleotides before the end of MDV-1 (+) RNA. The resulting transcripts, though lacking the natural 3′-terminal dinucleotide, CpA-OH, serve as excellent templates for exponential replication by Qβ replicase. A full description of the construction and use of this plasmid will be published elsewhere (13).

### Plasmid Containing a Polylinker within the MDV-1 cDNA Sequence

The PpuM I-BstE II fragment of the MDV-1 cDNA sequence in pT7-MDV DNA was replaced with the corresponding PpuM I-BstE II fragment of a modified MDV-1 cDNA that contained a unique Xba I recognition sequence in place of the only Hinf I recognition sequence in MDV-1 cDNA (21). A synthetic DNA fragment (prepared by annealing dCTAGATCTCGAGGCCTG to dCTAGCAGGCCTCGAGAT) was then cloned into this Xba I site, converting it into a polylinker possessing unique restriction sites for Xba I, Bgl II, Xho I, and Stu I. This plasmid, designated pT7-MDV-poly, is diagrammed in Figure 1. The RNA synthesized from this plasmid is designated MDV-poly. Recombinant RNAs can be generated by inserting any heterologous DNA sequence into one of the unique restriction sites in the polylinker of pT7-MDV-poly, and then utilizing the resulting plasmid as a template for transcription by T7 RNA polymerase.

### Plasmids for Synthesizing Recombinant Probes by Transcription

pT7-MDV-fal-un was constructed by inserting a synthetic probe sequence (prepared by annealing dTCGAGACTAACATAGGTCTTAACTTGACTAACA to dTCGATGTTAGTCAAGTTAAGACCTATGTTAGTC) into the Xho I site of the polylinker sequence in pT7-MDV-poly. pT7-MDV-fal-st was constructed by inserting the related synthetic probe sequence (prepared by annealing dTCGAGACTAACATAGGTCTAACTTGTTAGTCA to dTCGATGACTAACAAGTTAAGACCTATGTTAGTC) into the site in pT7-MDV-poly. Both probe sequences were complementary to the unique 21-base-pair sequences motif that is repeated over 1,000 times in P. falciparum DNA (14-16). The specific sequence used herein was determined by sequencing cloned repetitive DNA isolated from strain FCR-3/Gambia.

### Nucleotide Sequence Analysis

The nucleotide sequence in the recombinant region of each plasmid was determined by the chain termination procedure (22), utilizing 7-deaza-deoxyguanosine 5′-triphosphate (Boehringer Mannheim) in place of deoxyguanosine 5′-triphosphate (23), and [³⁵S]deoxycytidine 5′(α-thio) triphosphate (New England Nuclear) as label (24). Sequencing reactions were carried out on total plasmid DNA (25), utilizing a 20-nucleotide primer (Pharmacia) that was complementary to the T7 promoter sequence (26).

### Transcription

Plasmids were isolated from bacteria by the method of Holmes and Quigley (27) and purified by gel filtration chromatography (28) on Sephacryl S-1000 (Pharmacia). Plasmid DNA was then digested with Sma I or Stu I. Transcription was carried out according to a modification of the protocol of Axelrod and Kramer (29): I »g of linearized DNA was incubated with 80 units of T7 RNA polymerase for 3 hr at 37°C in 40 »l of 400 »M ATP, 400 »M [α-³²P] CTP, 400 »M GTP, 400 »M UTP, 50 mM Tris-HCl (pH 8.0), 12mM MgCl₂, 5 mM dithiothreitol (freshly prepared), 100 »g/ml bovine serum albumin, 4 mM spermidine, and 1 unit/»l of the ribonuclease inhibitor, RNasin (Promega Biotec). The [³²P] transcripts were incubated with 0.1 »g/»l of ribonuclease-free deoxyribonuclease I (30) to destroy the template DNA, and then purfied by phenol/chloroform/isoamyl alcohol extraction, followed by precipitation with ethanol. The concentration of each RNA was determined from its specific radioactivity. The size and homogeneity of the RNA was determined by electrophoresis through 6% polyacrylamide slab gels in the presence of 7 M urea (31). The RNA bands were visualized by silver staining (32).

### Replication

Between 1.4 x 10⁻¹⁸g and 1.4 x 10⁻¹⁰g of transcripts (depending on the reaction) were incubated with 2.4 x 10⁻⁶g of Qβ replicase at 37°C in 25 »l of 400 »M ATP, 400 »M [α-³²P]CTP, 400 »M GTP, 400 »M UTP, 14 mM MgCl₂, and 90 mM Tris-HCl (pH 7.5). Each reaction was sampled every 5 min. The amount of RNA in each 2-»l sample was determined by binding the [³²P]RNA to DE81 cellulose discs (Whatman), as described by Maxwell and his coworkers (33), and measuring the radioactivity on each disc with a scintillation counter. The size and homogeneity of the RNA in selected samples was determined by polyacrylamide gel electrophoresis. The [³²P]RNA bands were detected by autoradiography.

### Hybridization

Two different plasmids were used as targets. The first, pPFR6, was constructed by the insertion of a DNA fragment containing 45 tandem copies of the 21 base-pair repetitive sequence of P. falciparum DNA (strain FCR-3/Gambia) into the BamH I site of pUC13 DNA (Boehringer Mannheim). Fifteen of the 45 repeated sequences were identical to the inserted sequence in the recombinant RNA probes. The other repeated sequences contained microheterogeneities. The second plasmid, pUC13, served as a negative control for hybridization of the recombinant RNA probes. Each plasmid was linearized by digestion with BamH I, denatured by incubation in 0.4n NaOH for 60 sec at 42°C, and 0.5-»g or 1.0-»g aliquots were bound to a BA83 nitrocellulose membrane (Schleicher and Schuell), according to the dot-blot procedure of Kafatos and his coworkers (34). The membrane was cut into sections, each containing a duplicate pair of pPFR6 and pUC13 dot-blots. Each membrane section was prehybridized for 3 hr at 37°C in 5X SSPE (5X SSPE is 900 mM NaCl, 50 mM sodium phosphate (pH 7.4), and 5 mM EDTA), 2 mg/ml sodium dodecyl sulfate, 500 »g/ml heparin (Sigma), and 20% formamide. Each membrane section was then incubated for 4 hrs at 25°C with between 23 ng and 150 ng of the [³²P]RNA to be tested and 10 »g of unlabelled E. coli tRNA carrier (Boehringer Mannheim) dissolved in 2 ml of the prehybridization buffer. The following RNAs were tested: MDV-poly, MDV-fal-un, MDV-fal-st, and truncated versions of each (transcribed from plasmids cleaved at the Stu I site). After hybridization, the membranes were washed three times (15 min/wash) at 25°C with a solution containing 4X SSPE, 2 mg/ml sodium dodecyl sulfate, and 400 »g/ml heparin, followed by two 10-min washes at 25°C in 1X SSPE, and a 12-min wash at 37°C in 1X SSPE. The hybridized RNA was detected by autoradiography.

### Replication of Recombinant RNAs after Hybridization

To study the replicability of bound RNA, MDV-fal-un RNA hybridized to dot-blots was eluted by boiling in 200 »l of water for 60 sec. The integrity of the eluted RNA was analyzed by polyacrylamide gel electrophoresis. The replicability of the eluted RNA was determined by initiating a Qβ replicase reaction with 0.1 »l of the eluant. This reaction was followed kinetically, and the identity of the product RNA was determined by polyacrylamide gel electrophoresis. In addition, individual hybridized dot-blots were added directly to Qβ replicase reactions to see whether bound RNA could serve as a template. These reactions contained 100 »g/ml bovine serum albumin to preclude the possibility that Qβ replicase would bind to the nitrocellulose membrane.

### Preparation of Hybridization Reactions

Eight different 70-»liter hybridization reactions were prepared containing the following number of molecules of a transcript of the HIV-1 pol region: 2.5x10⁸, 2.5x10⁷, 2.5x10⁶, 2.5x10⁵, 2.5x10⁴, 2.5x10³, 2.5x10², and 0. 5x10⁸ molecules of a recombinant RNA (shown in Figure 2A) containing a probe sequence that is complementary to a conserved section of the pol region of HIV-1 mRNA was added to each of the eight reactions and hybridized to the target molecules in the presence of 2.5 M guanidine thiocyanate. The resulting probe-target hybrids were isolated by three cycles of reversible target capture on magnetic beads, as described on page 36 of the proposal. The recombinant-RNA probes were then released from the isolated hybrids by incubation in 50 »liters of a salt-free buffer at 37°C. 40 »liters of each of these eight solutions was used to initiate eight corresponding 120-»liter Qβ replicase amplification reactions. These reactions were incubated in parallel. 5-»liters samples of each of the eight reactions were withdrawn at two-minute intervals between 6 and 28 minutes. The concentration of magnesium in these reactions (7 mM) was half of that usually used, because we recently discovered that at lower magnesium concentrations the rate of replication of contaminating MDV-1 RNA is suppressed compared with the rate of replication of recombinant RNA. A portion of the RNA contained in each of the 96 samples was analyzed by acrylamide gel electrophoresis and shown to contain only recombinant RNA, confirming the utility of using a lower magnesium concentration. Finally, a portion of the RNA contained in each of the 96 samples (corresponding to a 1-»liter aliquot of the amplification reaction) was precipitated in 3.5% phosphoric acid and bound to a nylon membrane in a 96-position dot-blot format.

### Experimental Results

### Design of the Replicatable Probes

One of the goals of the present invention was to construct an RNA that would serve both as a specific probe for P. falciparum DNA and as a template for exponential amplification by Qβ replicase. MDV-1 RNA was chosen as the parent molecule because modified MDV-1 RNAs can be synthesized by transcription from recombinant plasmids (13). MDV-1 RNA contains many stable secondary structures (35-37), and these secondary structures are required for replication (38-40). The site which was chosen for inserting probe sequences into MDV-1 RNA was located on the exterior of the molecule (12), where the insert was less likely to disturb the structure, and therefore less likely to interfere with replication. In selecting the sequence of the probe two main concerns had to be reconciled: 1) the inserted sequence might have to assume a single-stranded conformation for it to hybridize to its target sequence; and 2) the inserted sequence might have to form secondary structures, otherwise the product and the template might form a lethal duplex during replication (40). Because these two concerns imposed contradictory constraints on the design of the inserted sequence, two recombinant RNAs were prepared each containing a different probe sequence. The first recombinant, MDV-fal-un RNA, was likely to possess an unstructured probe region, according to a computer analysis of its sequence (41). The second recombinant, MDV-fal-st RNA, differed from MDV-fal-un RNA in a 5-nucleotide region. As a consequence, it was likely to form a more stable secondary structure in the region containing the probe. Figure 2 shows the nucleotide sequence and probable secondary structure (41) of MDV-fal-un RNA, MDV-fal-st RNA, and MDV-poly RNA.

### Replication of the Recombinant RNAs

Four different RNAs were prepared by transcription in vitro: MDV-1, MDV-poly, MDV-fal-un, and MDV-fal-st. Electrophoretic analysis of these transcripts, as illustrated in Figure 3(A), demonstrated that the recombinants that contained probe sequences could be distinguished easily from the other transcripts by their relative mobility. The transcripts were then isolated from the reaction mixture and used as templates for the synthesis of additional RNA by Qβ replicase. Kinetic analysis of the amount of RNA synthesized in the Qβ replicase reactions, as shown in Figure 3(B), demonstrated that both the structured and the unstructured recombinant RNAs were excellent templates for exponential replication. Furthermore, as shown in Figure 3(C), electrophoretic analysis of the RNA synthesized in each Qβ replicase reaction, showed that the products were homogeneous replicates of the original transcripts.

To compare the rate of replication of recombinant RNA to the rate of replication of MDV-1 RNA, Qβ replicase reactions were initiated with mixtures of MDV-fal-st (+) RNA and MDV-1 (+) RNA. The reactions were incubated for 20 minutes, permitting the synthesis of approximately ten million copies of each RNA. The RNA products of the reactions were analyzed by polyacrylamide gel electrophoresis. The results are shown in Table 1 and indicate that, despite recombinant RNA being 55 nucleotides longer than the MDV-1 RNA from which it was derived, it is amplified at essentially the same rate by Qβ replicase.

**Table 1**

| Replication of Mixtures of MDV-1 RNA and Recombinant RNA | | | |
|---|---|---|---|
| Initial RNA (fg) | | Product RNA (ng) | |
| MDV-1 | MDV-fal-st | MDV-1 | MDV-fal-st |
| 1 | 1 | 42 | 54 |
| 10 | 1 | 115 | 13 |
| 1 | 10 | 17 | 149 |

### Exponential Amplification after Serial Dilution

Because the sensitivity of bioassays employing replicatable probes will depend on how few recombinant molecules can serve as templates for the synthesis of an easily detectable quantity of product RNA, a series of Qβ replicase reactions were initiated with decreasing amounts of MDV-fal-un RNA. The results of this series of reactions are shown in Figure 4 and demonstrate that a reaction initiated with as few as 1,000 molecules of recombinant RNA (0.14 fg) produced 129 nanograms of recombinant RNA product after 30 minutes of synthesis, which is a one-billion-fold amplification. Virtually identical kinetic results were obtained in a parallel series of reactions initiated with MDV-fal-st RNA, indicating that the relative lack of secondary structure in the probe region of MDV-fal-un RNA had no discernible effect on replication.

Electrophoretic analysis showed that the product of the reaction to which no exogenous template had been added was MDV-1 RNA. This analysis is illustrated in the inset of Figure 4. This was expected, because the method which was used for isolating Qβ replicase (17) does not eliminate all of the contaminating MDV-1 RNA molecules (5). Based on the time it took for this reaction to achieve saturation (when the number of RNA molecules equals the number of active replicase molecules), it is calculated that only 40 molecules of MDV-1 RNA were present initially. This was confirmed by the following results: when a reaction was initiated with 100 molecules of MDV-fal-un RNA, the 25-minute product contained 30% MDV-1 RNA; and a reaction initiated with only 10 molecules of MDV-fal-un RNA contained mostly MDV-1 RNA. Had Qβ replicase that had been freed of all contaminating RNA (43) been used instead, it would have been expected that only recombinant RNA would have been synthesized.

### Hybridization of the Recombinant RNAs

Each of the recombinant RNAs was shown to bind specifically to denatured plasmids containing a known quantity of P. falciparum target sequences (Figure 5). Neither recombinant RNA bound to control plasmids that lacked P. falciparum sequences. Furthermore, MDV-poly RNA, which does not contain a probe sequence, did not bind to either plasmid. Taken together, these results demonstrate that both recombinant RNAs bind specifically to their targets and that, under the hybridization conditions employed, the presence of structure in the probe region has little effect on hybridization.

In another experiment, truncated versions of each recombinant probe were prepared by transcription from plasmids that had been linearized by digestion with Stu I, instead of Sma I. These transcripts were 111-nucleotides long, and contained 63 nucleotides from the 5' end of MDV-1 (+) RNA and 48 nucleotides from the insert region, including the entire probe sequence. Under the hybridization conditions employed, no difference could be observed between the hybridization of the truncated RNAs and the hybridization of the full-length RNAs. This result demonstrates that the constrained topology of the MDV-1 domain (44) that surrounds the probe region in the full-length molecule has little effect on the ability of the probe to hybridize to its target.

### Replication of Recombinant RNAs after Hybridization

Additional experiments were carried out to determine whether hybridized recombinant RNAs retain their ability to serve as templates for Qβ replicase after all the manipulations that occur during filter hybridization. MDV-fal-un RNA that had been hybridized to plasmids containing P. falciparum sequences was eluted from the dot-blot by a brief heating step. Electrophoretic analysis of this RNA showed that intact recombinant molecules had been released. A portion of the eluted RNA was then tested to see whether it could serve as a template for Qβ replicase. A ten-million-fold amplification occurred in 20 minutes. Electrophoretic analysis of the product of this reaction showed that it was pure MDV-fal-un RNA. From the results of these experiments it was estimated that at least 10 percent of the eluted RNA had retained its biological activity. Another experiment was performed to see whether recombinant probes could serve as templates for Qβ replicase while still bound to dot-blots. Membrane fragments containing hybridized MDV-fal-un RNA were incubated directly with Qβ replicase. Reactions containing membranes with RNA hybridized to target sequences synthesized more recombinant RNA than reactions containing membranes that lacked target sequences. These preliminary results suggest that a special release step may not be necessary to initiate the replication of hybridized recombinant RNAs.

### Results of Hybridization Reactions

The results are shown in Figure 7.

### Experimental Discussion

In most bioassays that employ molecular probes, the amount of probe bound is proportional to the number of targets. Amplification schemes, such as those employing enzymatic reporter groups (45) or polymerase chain reactions (46-48), enable a small number of targets to be detected. However, the relationship between the size of the amplified signal and the number of targets is still linear. The unique kinetic characteristics of Qβ replicase reactions should permit the design of bioassays in which the size of the amplified signal is linearly proportional to the logarithm of the number of targets. Figure 6 utilizes date from the experiment shown in Figure 4 to illustrate this relationship: the logarithm of the number of recombinant RNA molecules initially present in each reaction was plotted against the amount of RNA synthesized in 25 minutes (by which time each reaction had completed the exponential phase of synthesis). The amount of recombinant RNA initially added to those reactions can be thought of as representing the amount of probe that would have been bound to targets had this been an actual assay, and the amount of amplified RNA at 25 minutes simulates the signal that would have been detected. The results show that the size of the amplified signal would be in the hundred-nanogram range, whether the number of targets was as small as one thousand or as large as one billion. Thus, bioassays employing amplification by Qβ replicase should be able to detect targets over a range of at least six orders of magnitude.

Theoretically, assays employing Qβ replicase should be extremely sensitive, because such reactions can be initiated with as little as one molecule of template RNA (10). In practice, the sensitivity of assays will be limited by the level of persistence of nonspecifically bound probes. In the experiments described above it was found that 600,000 molecules of MDV-fal-un RNA had bound nonspecifically to nitrocellulose dot-blots that contained 1.5 »g of pUC13 DNA. It is apparent that a different hybridization technique will be required to test the limits of sensitivity of assays employing replicatable probes. There are a number of promising methods that should result in a markedly reduced background, including those where hybridization occurs in solution (49-51) and those that employ "sandwich" hybridization techniques (52,53). By combining an effective background reduction technique with the enormous amplification inherent in the use of replicatable probes, it should be possible to develop assays that can detect a few hundred molecules of target. This would permit the detection of relatively rare RNA or DNA molecules in a research sample and the detection of even a single infectious agent in a clinical sample.

Replicatable recombinant RNAs containing a variety of inserted sequences have recently been constructed. This leads one to believe that, by choosing appropriate inserts, recombinant RNA probes can be prepared that will be able to detect the nucleic acid of any virus, bacterium, or eukaryotic parasite.

Amplified recombinant RNAs contain a sequence (the probe) that identifies which target was detected. It should therefore be possible to design diagnostic assays that utilize a mixture of recombinant RNAs, each containing a probe sequence specific for the genome of a different infectious agent. After amplification with Qβ replicase, the RNA population would contain replicates of only those probes that had bound to their targets. Subsequent hybridization of these amplified RNAs to a membrane containing an ordered array of DNA dot-blots complementary to each of the probes would permit the simultaneous identification of several different organisms in the same sample.

The density of each spot in Figure 7 indicates the amount of RNA that is present at each time point. Although no RNA can be seen during the early time points of each reaction, this is the period in which the RNA population is increasing exponentially. The RNA becomes visible at about the same time that the number of RNA molecules equals the number of active replicase molecules (the "saturation point"). Consequently, the amount of RNA in the visible time points increases linearly. The results show that the time at which the saturation point occurs in each amplification reaction is a function of the number of target molecules that were originally present in the corresponding hybridization reaction. The less target molecules that were present, the less the number of probes that were bound to targets, and thus, the less the number of probes that were available to initiate the amplification reaction. This result demonstrates that an automated assay could be developed, in which a detection device (based, perhaps, on the fluorescence of an RNA ligand, such as ethidium bromide) could be used to determine the time of saturation. The number of probes originally present in the amplification reaction (which would be proportional to the number of targets in the sample) could then be calculated from the time it took to reach saturation. The results also demonstrate an alternative way to determine the number of targets present in the sample. The amount of RNA present at a particular time point, after all reactions have achieved saturation (for example, at 24 minutes), is linearly proportional to the logarithm of the number of probes originally present in each amplification reaction (see also Figure 6).

Finally, the results of this improved assay show that the current limit of sensitivity is between 2,500 and 25,000 molecules of target. Reactions initiated with probes released from hybridization reactions that contained a small number of targets gave virtually indistinguishable results. Since the RNA that grew out of these reactions was recombinant RNA, the released RNA must have mainly consisted of non-specifically hybridized probes that were not removed during the hybrid isolation procedure.

### References

- 1.: Gillespie, D. and Spiegelman, S. (1965) J. Mol. Biol. 12, 829-842.
- 2.: Chu, B.C.F., Kramer, F.R. and Orgel, L.E. (1986) Nucleic Acids Res. 14, 5591-5603.
- 3.: Haruna, I. and Spiegelman, S. (1965) Proc. Natl. Acad. Sci. USA 54, 579-587.
- 4.: Haruna, I. and Spiegelman, S. (1965) Science 150, 884-886.
- 5.: Kramer, F.R., Mills, D.R., Cole, P.E., Nishihara, T. and Spiegleman, S. (1974) J. Mol. Biol. 89, 719-736.
- 6.: Weissmann, C., Felix, G. and Slor, H. (1986) Cold Spring Harbor Symp. Quant. Biol. 33, 83-100.
- 7.: Spiegelman, S., Pace, N.R., Mills, D.R., Levisohn, R., Eikhom, T.S., Taylor, M.M., Peterson, R.L. and Bishop, D.H.L. (1968) Cold Spring Harbor Symp. Quant. Biol. 33, 101-124.
- 8.: Dobkin, C., Mills, D.R., Kramer, F.R. and Spiegelman, S. (1979) Biochemistry 18, 2038-2044.
- 9.: Haruna, I. and Spiegelman, S. (1965) Proc. Natl. Acad. Sci. USA 54, 1189-1193.
- 10.: Levisohn, R. and Spiegelman, S. (1969) Proc. Natl. Acad. Sci. USA 60, 866-872.
- 11.: Kacian, D.L., Mills, D.R., Kramer, F.R. and Spiegelman, S. (1972) Proc. Natl. Acad. Sci. USA 69, 3038-3042.
- 12.: Miele, E.A., Mills, D.R. and Kramer, F.R. (1983) J. Mol. Biol. 171, 281-295.
- 13.: Lizardi, P.M., Gueria, C.E., Lomeli, H., Axelrod, V.D., Tussie-Luna, I., and Kramer, F.R. (1988) Bio/Technology, 6(10): 1197-1202
- 14.: Franzen, L., Westin, G., Shabo, R., Aslund, L., Perlmann, H., Persson, T., Wigzell, H. and Pettersson, U. (1984) Lancet 1, 525-528.
- 15.: Aslund, L., Franzen, L., Westin, G., Persson, T., Wigzell, H. and Pettersson, U. (1985) J. Mol. Biol. 185, 509-516.
- 16.: Zolg, J.W., Andrade, L.E. and Scott, E.D. (1987) Mol. Biochem. Parasitol. 22, 145-151.
- 17.: Eoyang, L. and August, J.T. (1971) in Cantoni, G.L. and Davis, D.R. (eds) Procedures in Nucleic Acid Research, Harper and Row, New York, Vol. 2, pp. 829-839.
- 18.: Gait, M.J. (1984) Oligonucleotide Synthesis, IRL Press, Oxford.
- 19.: Matthes, H.W.D., Zenke, W.M. Grundstrom, T., Staub, A., Wintzerith, M. and Chambon, P. (1984) EMBO J. 3, 801-805.
- 20.: Lo, K-M., Jones, S.S., Hackett, N.R. and Khorana, H.G. (1984) Proc. Natl. Acad. Sci. USA 81 2285-2289.
- 21.: Bausch, J.N. Kramer, F.R., Miele, E. A., Dobkin, C. and Mills, D.R. (1983) J. Biol. Chem. 258, 1978-1984.
- 22.: Sanger, F., Nicklen, S. and Coulson, A.R. (1977) Proc. Natl. Acad. Sci. USA 74, 5463-5467.
- 23.: Mizusawa, S., Nishimura, S. and Seela, F. (1986) Nucleic Acids Res. 14, 1319-1324.
- 24.: Biggin, M.D., Gibson, T.J. and Hong, G.F. (1983) Proc. Natl. Acad. Sci. USA 80, 3963-3965.
- 25.: Wallace, R.B., Johnson, M.J. Suggs, S.V., Miyoshi, K., Bhatt, R, and Itakura, K. (1981) Gene 16, 21-26.
- 26.: Osterman, H.L and Coleman, J. E. (1981) Biochemistry 20, 4885-4892.
- 27.: Holmes, D.S. and Quigley, M. (1981) Anal Biochem. 114, 193-197.
- 28.: Bywater, M., Bywater, R. and Hellman, L. (1983) Anal. Biochem. 132, 219-224.
- 29.: Axelrod, V.D. and Kramer, F.R. (1985) Biochemistry 24, 5716-5723.
- 30.: Tullis, R.H. and Rubin, H. (1980) Anal. Biochem. 107, 260-264.
- 31.: Maniatis, T., Jeffrey, A. and van deSande, H. (1975) Biochemistry 14, 3787-3794.
- 32.: Sammons, D.W., Adams, L.D. and Nishizawa, E.E. (1981) Electrophoresis 2, 135-141.
- 33.: Maxwell, I.H., Van Ness, J. and Hahn, W.E. (1978) Nucleic Acids Res. 5, 2033-2038.
- 34.: Kafatos, F.C. Jones, C.W. and Efstratiadis, A., (1979) Nucleic Acids Res. 7, 1541-1552.
- 35.: Klotz, G., Kramer, F.R., and Kleinschmidt, A.K. (1980) Electron Microscopy 2, 530-531.
- 36.: Mills, D.R., Kramer, F.R., Dobkin, C., Nishihara, T. and Cole, P.E. (1980) Biochemistry 19, 228-236.
- 37.: Kramer, F.R. and Mills, D.R. (1981) Nucleic Acids Res. 19, 5109-5124.
- 38.: Mills, D.R., Dobkin, C. and Kramer, F.R. (1978) Cell 15, 541-550.
- 39.: Nishihara, T., Mills, D.R. and Kramer, F.R. (1983) J. Biochem. 93, 669-674.
- 40.: Priano, C., Kramer, F.R. and Mills, D.R. (1987) Cold Spring Harbor Symp. Quant. Biol. 52, 321-330.
- 41.: Zuker, M. and Stiegler, P. (1981) Nucleic Acids Res. 9, 133-148.
- 42.: Mills, D.R., Kramer, F.R. and Spiegelman, S. (1973) Science 180, 916-927.
- 43.: Hill, D. and Blumenthal, T. (1983) Nature 301, 350-352.
- 44.: Biebricher, C.K., Diekmann, S. and Luce, R. (1982) J. Mol. Biol. 154, 629-648.
- 45.: Leary, J.J., Brigati, D.J. and Ward, D.C. (1983) Proc. Natl. Acad. Sci. USA 80, 4045-4049.
- 46.: Saiki, R.K., Scharf, S., Faloona, F., Mullis, K.B., Horn, G.T., Erlich, H.A. and Arnheim, N. (1985) Science 230, 1350-1354.
- 47.: Saiki, R.K., Gelfand, D.H., Stoffel, S., Scharf, S.J., Higuchi, R., Horn, G.T., Mullis, K.B. and Erlich, H.A. (1988) Science 239, 487-491.
- 48.: Erlich, H.A., Gelfand, D.H. and Saiki, R.K. (1988) Nature 331, 461-462.
- 49.: Kohne, D.E, Levison, S.A. and Byers, M.J. (1977) Biochemistry 16, 5329-5341.
- 50.: Pellegrino, M.G., Lewin, M., Meyer, III, W.A. Lanciotti, R.S., Bhaduri-Hauck, L., Volsky, D.J., Sakai, K., Folks, T.M. and Gillespie, D., (1987) BioTechniques 5, 452-459.
- 51.: Urdea, M.S., Running, J.A. Horn, T., Clyne, J., Ku, L. and Warner, B. D. (1987) Gene 61, 253-264.
- 52.: Palva, A. and Ranki, M. (1985) Clin. Lab. Med. 5, 475-490.
- 53.: Syvanen, A.-C., Laaksonen, M. and Soderlund, H. (1986) Nucleic Acids Res. 14, 5037-5048.
- 54.: Kutateladze, T.V., Axelrod, V.D. Gorbulve, V.G., Belzhelarskaya, S.N. and Vartikyan, R.M. (1979) Anal. Biochem. 100, 129-135.
- 55.: Matthews, J.A., Batki, A., Hynds, C. and Kricka, L.J. (1985) Anal. Biochem. 151, 205-209.
- 56.: Forster, A.C., Melnnes, J.L. Skingle, D.C. Symons, R.H. (1985), Nucleic Acids Res. 13(3), 745-761.
- 57.: Cosstick, R., McLaughlin, L.W. and Eckstein, F. (1984) Nucleic Acids Res. 12, 1791-1810.
- 58.: Dahlberg, A.E., Dingman, C. W. and peacock, A.C, (1969) J. Mol. Biol. 41, 139-147.
- 59.: Dingman, C.W. and Peacock, A.C., (1968) Biochemistry, 7, 2038-2044.
- 60.: Igloi, G.L. (1983) Anal. Biochem. 134, 184-188.
- 61.: Sharop, P.A., Sugden, B. and Sambrook, J. (1973) Biochemistry 12, 3055-3063.
- 62.: Bailey, J.M. and Davidson, N. (1976) Anal. Biochem. 70. 75-85.
- 63.: Oi, V. T., Glazer, A.N. and Stryer, L. (1982) J. Cell. Biol. 93, 981-986.
- 64.: Martin, W.J. (1983) Am. J. of Med. July 28, Suppl. 119-123.
- 65.: Hughes, W.M., Datta, M. (1983), Nature 302: 725-726.
- 66.: Spies, T., Laufs, R., Riess, S.-C. (1983), J. Bacteriol. 55: 839-846.

## Claims

1. A replicatable and hybridizable recombinant single-stranded RNA probe molecule comprising:
(a) a recognition sequence for the binding of an RNA-directed RNA polymerase;
(b) a sequence required for the initiation of product strand synthesis by the polymerase; and
(c) a heterologous RNA sequence complementary to a specific nucleic acid sequence of an infectious agent and inserted at a specific site in the internal region of the recombinant molecule, such that the heterologous sequence of the probe molecule may have single-stranded conformation for hybridization with a target sequence and may form secondary structures for efficient replication.

2. A recombinant RNA probe molecule of claim 1, wherein the recognition sequence for the binding of an RNA-directed RNA polymerase is in an internal region of the molecule.

3. A recombinant RNA probe molecule of claim 1, wherein the sequence required for the initiation of product strand synthesis is a cytidine-rich 3'-terminal sequence.

4. A recombinant RNA probe molecule of claim 1, wherein the RNA-directed RNA polymerase is Qβ replicase.

5. A recombinant RNA probe molecule of claim 1, wherein the molecule is a variant RNA template for Qβ replicase or a mutant thereof.

6. A recombinant RNA probe molecule of claim 5, wherein the variant RNA template is MDV-1 RNA or a mutant thereof.

7. A recombinant RNA probe molecule of claim 6, wherein the MDV-1 RNA is MDV-1 (+) RNA.

8. A recombinant RNA probe molecule of claim 6, wherein the MDV-1 RNA is MDV-1 (-) RNA.

9. A recombinant RNA probe molecule of claim 7, wherein the heterologous sequence of interest is inserted between nucleotides 63 and 64.

10. A recombinant RNA probe molecule of claim 1, wherein the molecule is a transcript derived from a recombinant plasmid by incubation with a DNA-directed RNA polymerase.

11. A recombinant RNA probe molecule of claim 10, wherein the molecule is a variant RNA template for Qβ replicase or a mutant thereof.

12. A recombinant RNA probe molecule of claim 11, wherein the molecule is MDV-1 RNA or a mutant thereof.

13. A recombinant RNA probe molecule of claim 12, wherein the MDV-1 RNA is MDV-1 (+) RNA.

14. A recombinant RNA probe molecule of claim 12, wherein the MDV-1 RNA is MDV-1 (-) RNA.

15. A recombinant RNA probe molecule of claim 13, wherein the heterologous sequence is inserted between nucleotides 63 and 64.

16. A recombinant RNA probe molecule of claim 1, wherein the infectious agent is a virus.

17. A recombinant RNA probe molecule of claim 1, wherein the infectious agent is a viroid or virusoid.

18. A recombinant RNA probe molecule of claim 1, wherein the infectious agent is a prokaryote.

19. A recombinant RNA probe molecule of claim 18, wherein the prokaryote is a bacterium.

20. A recombinant RNA probe molecule of claim 1, wherein the infectious agent is a eukaryote.

21. A recombinant RNA probe molecule of claim 20, wherein the eukaryote is a parasitic protozoan.

22. A recombinant RNA probe molecule of claim 21, wherein the parasitic protozoan causes malaria.

23. A replicatable and hybridizable recombinant single-stranded RNA probe molecule comprising:
(a) a recognition sequence for the binding of an RNA-directed RNA polymerase;
(b) a sequence required for the initiation of product strand synthesis by the polymerase; and
(c) a heterologous RNA sequence, other than the sequence 5'AGUCAGGCACCGUGUAUGAAAUCUAACAAU-3', complementary to a specific gene sequence or portion thereof inserted at a specific site in the internal region of the recombinant molecule, such that the heterologous sequence of the probe molecule may have single-stranded conformation for hybridization with a target sequence and may form secondary structures for efficient replication.

24. A recombinant RNA probe molecule of claim 23, wherein the specific gene sequence is an allele or portion thereof.

25. A method for determining the presence or concentration of a specific nucleic acid sequence of an infectious agent in a sample, comprising the steps of:
(a) forming a specific complex between the recombinant RNA probe molecule of claim 1 and the specific nucleic acid sequence of an infectious agent, by incubating the sample with the recombinant RNA probe molecules under suitable conditions and for a sufficient period of time to permit complementary nucleotide sequences to hybridize;
(b) removing unhybridized recombinant RNA probe molecules from the reaction mixture;
(c) incubating the reaction mixture with an RNA-directed RNA polymerase capable of synthesizing additional copies of the recombinant RNA probe molecules that are hybridized to the specific nucleic acid sequence of the infectious agent; and
(d) detecting the recombinant RNA probe molecules synthesized in step (c), thereby determining the presence or concentration of the specific nucleic acid sequence of the infectious agent.

26. A method for determining the presence or concentration of a specific gene sequence or portion thereof in a sample, comprising the steps of:
(a) forming a specific complex between the recombinant RNA probe molecule of claim 23 and the specific gene sequence or portion thereof, by incubating the sample with the recombinant RNA probe molecules under suitable conditions and for a sufficient period of time to permit complementary nucleotide sequences to hybridize;
(b) removing unhybridized recombinant RNA probe molecules from the reaction mixture;
(c) incubating the reaction mixture with an RNA-directed RNA polymerase capable of synthesizing additional copies of the recombinant RNA probe molecules that are hybridized to the specific gene sequence or portion thereof; and
(d) detecting the recombinant RNA probe molecules synthesized in step (c), thereby determining the presence or concentration of the specific gene sequence or portion thereof.

27. A method of claim 25, wherein the specific nucleic acid sequence of the infectious agent in the sample is bound to a solid support.

28. A method of claim 26, wherein the specific gene sequence or portion thereof in the sample is bound to a solid support.

29. A method of claim 27 or 28, wherein the solid support is a nitrocellulose or nylon membrane.

30. A method of claim 25, wherein in step (a) the specific nucleic acid sequence of the infectious agent and the recombinant RNA probe molecule are in solution.

31. A method of claim 26, wherein in step (a) the specific gene sequence or portion thereof and the recombinant RNA probe molecule are in solution.

32. A method of claim 30, wherein in step (b) the hybridized recombinant RNA probe molecules are separated from the unhybridized probe molecules through the capture of the specific nucleic acid sequence of the infectious agent onto a solid support.

33. A method of claim 31, wherein in step (b) the hybridized recombinant RNA probe molecules are separated from the unhybridized probe molecules through the capture of the specific gene sequence or portion thereof onto a solid support.

34. A method of claim 25 or 26 wherein detecting is carried out by the incorporation of radioactively labelled ribonucleoside 5'-triphosphate precursors into the recombinant RNA products.

35. A method of claim 25 or 26 wherein detecting is carried out by the incorporation of chemically modified ribonucleoside 5'-triphosphate precursors into the recombinant RNA products.

36. A method of claim 35, wherein the chemically modified ribonucleoside 5'-triphosphate precursors are biotinylated.

37. A method of claim 35, wherein the chemically modified ribonucleoside 5'-triphosphate precursors are fluorescent.

38. A method of claim 25 or 26, wherein detecting is carried out by the binding of RNA-specific chromogenic or fluorogenic dyes to the recombinant RNA products.

39. A method of claim 25 or 26, wherein detecting is carried out by physical methods.

40. A method of claim 25 or 26, wherein in step (c) the RNA-directed RNA polymerase is Qβ replicase.

41. A method of claim 25 or 26, wherein the time of incubation in step (c) is sufficiently short so that the number of recombinant RNA product strands does not exceed the number of polymerase molecules, with the result that the number of recombinant RNA product molecules is linearly proportional to the number of recombinant RNA probe molecules originally hybridized.

42. A method of claims 25 or 26, wherein the time of incubation in step (c) is sufficiently long so that the number of recombinant RNA product strands exceeds the number of polymerase molecules, with the result that the number of recombinant RNA product molecules is proportional to the logarithm of the number of recombinant RNA probe molecules originally hybridized.

## Patentansprüche

1. Replizierbares und hybridisierbares rekombinantes einsträngiges RNA-Sondenmolekül, umfassend:
(a) eine Erkennungssequenz zum Binden einer auf RNA gerichteten RNA-Polymerase;
(b) eine für die Initiierung einer Produktstrangsynthese durch die Polymerase erforderliche Sequenz und
(c) eine zu einer spezifischen Nucleinsäuresequenz eines infektiösen Mittels komplementäre heterologe RNA-Sequenz, die derart an einer spezifischen Stelle in die interne Region des rekombinanten Moleküls eingefügt ist, daß die heterologe Sequenz des Sondenmoleküls eine einzelsträngige Konformation zur Hybridisierung mit einer Targetsequenz aufweisen und sekundäre Strukturen für eine effiziente Replikation ausbilden kann.

2. Rekombinates RNA-Sondenmolekül nach Anspruch 1, wobei die Erkennungssequenz zur Bindung einer auf RNA gerichteten RNA-Polymerase in einem internen Bereich des Moleküls liegt.

3. Rekombinantes RNA-Sondenmolekül nach Anspruch 1, wobei die für die Initiierung einer Produktstrangsynthese erforderliche Sequenz eine cytidinreiche 3'-terminale Sequenz ist.

4. Rekombinantes RNA-Sondenmolekül nach Anspruch 1, wobei die auf RNA gerichtete RNA-Polymerase Qβ-Replikase ist.

5. Rekombinantes RNA-Sondenmolekül nach Anspruch 1, wobei das Molekül eine eine Variante darstellende RNA-Matrize für eine Qβ-Replikase oder eine Mutante hiervon ist.

6. Rekombinantes RNA-Sondenmolekül nach Anspruch 5, wobei die eine Variante darstellende RNA-Matrize MDV-1 RNA oder eine Mutante hiervon ist.

7. Rekombinantes RNA-Sondenmolekül nach Anspruch 6, wobei die MDV-1 RNA MDV-1 (+) RNA ist.

8. Rekombinantes RNA-Sondenmolekül nach Anspruch 6, wobei die MDV-1 RNA MDV (-) RNA ist.

9. Rekombinantes RNA-Sondenmolekül nach Anspruch 7, wobei die interessierende heterologe Sequenz zwischen die Nucleotide 63 und 64 eingefügt ist.

10. Rekombinantes RNA-Sondenmolekül nach Anspruch 1, wobei das Molekül ein von einem rekombinanten Plasmid durch Inkubation mit einer auf DNA gerichteten RNA-Polymerase abgeleitetes Transkript ist.

11. Rekombinantes RNA-Sondenmolekül nach Anspruch 10, wobei das Molekül eine eine Variante darstellende RNA-Matrize für Qβ-Replikase oder eine Mutante hiervon ist.

12. Rekombinantes RNA-Sondenmolekül nach Anspruch 11, wobei das Molekül MDV-1 RNA oder eine Mutante hiervon ist.

13. Rekombinantes RNA-Sondenmolekül nach Anspruch 12, wobei die MDV-1 RNA MDV-1 (+) RNA ist.

14. Rekombinantes RNA-Sondenmolekül nach Anspruch 12, wobei die MDV-1 RNA MDV-1 (-) RNA ist.

15. Rekombinantes RNA-Sondenmolekül nach Anspruch 13, wobei die heterologe Sequenz zwischen die Nucleotide 63 und 64 eingefügt ist.

16. Rekombinantes RNA-Sondenmolekül nach Anspruch 1, wobei das infektiöse Mittel ein Virus ist.

17. Rekombinantes RNA-Sondenmolekül nach Anspruch 1, wobei das infektiöse Mittel ein Viroid oder ein Virusoid ist.

18. Rekombinantes RNA-Sondenmolekül nach Anspruch 1, wobei das infektiöse Mittel ein Prokaryont ist.

19. Rekombinantes RNA-Sondenmolekül nach Anspruch 18, wobei der Prokaryont ein Bakterium ist.

20. Rekombinantes RNA-Sondenmolekül nach Anspruch 1, wobei das infektiöse Mittel ein Eukaroyont ist.

21. Rekombinantes RNA-Sondenmolekül nach Anspruch 20, wobei der Eukaroyont ein parasitisches Protozon ist.

22. Rekombinantes RNA-Sondenmolekül nach Anspruch 21, wobei das parastisiche Protozon Malaria verursacht.

23. Replizierbares und hybridizierbares rekombinantes einsträngiges RNA-Sondenmolekül, umfassend:
(a) eine Erkennungssequenz zur Bindung einer auf RNA gerichteten RNA-Polymerase;
(b) einer für die Initiierung einer Produktstrangsynthese durch die Polymerase erforderliche Sequenz und
(c) eine von der Sequenz 5'-AGUCAGGCACCGUGUAUGAAAUCUAACAAU-3' verschiedene heterologe RNA-Sequenz, die zu einer spezifischen Gensequenz oder einem Teil hiervon komplementär ist und an einer speziellen Stelle im internen Bereich des rekombinanten Moleküls derart eingebaut ist, daß die heterologe Sequenz des Sondenmoleküls eine einzelsträngige Konformation zur Hybridisierung mit einer Targetsequenz aufweisen und Sekundärstrukturen für eine effiziente Replikation ausbilden kann.

24. Rekombinantes RNA-Sondenmolekül nach Anspruch 23, wobei die spezifische Gensequenz ein Allel oder ein Teil hiervon ist.

25. Verfahren zur Bestimmung der Anwesenheit oder Konzentration einer spezifischen Nucleinsäuresequenz eines infektiösen Mittels in einer Probe, das die folgenden Schritte umfaßt:
(a) Ausbilden eines spezifischen Komplexes zwischen dem rekombinanten RNA-Sondenmolekül nach Anspruch 1 und der speziellen Nucleinsäuresequenz eines infektiösen Mittels durch Inkubieren der Probe mit den rekombinanten RNA-Sondenmolekülen unter geeigneten Bedingungen und während einer ausreichenden Zeitdauer, um eine Hybridisierung komplementärer Nucleotidsequenzen zu gewährleisten;
(b) Entfernen nicht hybridisierter rekombinanter RNA-Sondenmoleküle aus dem Reaktionsgemisch;
(c) Inkubieren des Reaktionsgemisches mit einer auf RNA gerichteten RNA-Polymerase mit der Fähigkeit zur Synthese weiterer Kopien der rekombinanten RNA-Sondenmoleküle, die mit der spezifischen Nucleinsäuresequenz des infektiösen Mittels hybridisiert sind, und
(d) Nachweisen der in Stufe (c) synthetisierten rekombinanten RNA-Sondenmoleküle zur Bestimmung der Anwesenheit oder Konzentration der spezifischen Nucleinsäuresequenz des infektiösen Mittels.

26. Verfahren zur Bestimmung der Anwesenheit oder Konzentration einer spezifischen Gensequenz oder eines Teils hiervon in einer Probe, das die folgenden Schritte umfaßt:
(a) Ausbilden eines speziellen Komplexes zwischen dem rekombinanten RNA-Sondenmolekül nach Anspruch 23 und der spezifischen Gensequenz oder eines Teils hiervon durch Inkubieren der Probe mit den rekombinanten RNA-Sondenmolekülen unter geeigneten Bedingungen und während einer ausreichenden Zeitdauer, um eine Hybridisierung komplementärer Nucleotidsequenzen zu gewährleisten;
(b) Entfernen nicht hybridisierter rekombinanter RNA-Sondenmoleküle aus dem Reaktionsgemisch;
(c) Inkubieren des Reaktionsgemisches mit einer auf RNA gerichteten RNA-Polymerase mit der Fähigkeit zur Synthese weiterer Kopien der rekombinanten RNA-Sondenmoleküle, die mit der spezifischen Gensequenz oder eines Teils hiervon hybridisiert sind, und
(d) Nachweisen der in Stufe (c) synthetisierten rekombinanten RNA-Sondenmoleküle zur Bestimmung der Anwesenheit oder Konzentration der spezifischen Gensequenz oder eines Teils hiervon.

27. Verfahren nach Anspruch 25, wobei die spezifische Nucleinsäuresequenz des infektiösen Mittels in der Probe an einen festen Träger gebunden ist.

28. Verfahren nach Anspruch 26, wobei die spezifische Gensequenz oder ein Teil hiervon in der Probe an einen festen Träger gebunden ist.

29. Verfahren nach Anspruch 27 oder 28, wobei der feste Träger eine Nitrocellulose oder Nylonmembran ist.

30. Verfahren nach Anspruch 25, wobei in Stufe (a) die spezifische Nucleinsäuresequenz des infektiösen Mittels und das rekombinante RNA-Sondenmolekül in Lösung vorliegen.

31. Verfahren nach Anspruch 26, wobei in Stufe (a) die spezifische Gensequenz oder ein Teil hiervon und das rekombinante RNA-Sondenmolekül in Lösung vorliegen.

32. Verfahren nach Anspruch 30, wobei in Stufe (b) die hybridisierten rekombinanten RNA-Sondenmoleküle von den nicht hybridisierten Sondenmolekülen durch Einfangen der spezifischen Nucleinsäuresequenz des infektiösen Mittels auf einem festen Träger getrennt werden.

33. Verfahren nach Anspruch 31, wobei in Stufe (b) die hybridisierten rekombinanten RNA-Sondenmoleküöse von den nicht-hybridisierten Sondenmolekülen durch Einfangen der spezifischen Gensequenz oder eines Teils hiervon auf einem festen Träger getrennt werden.

34. Verfahren nach Anspruch 25 oder 26, wobei das Nachweisen durch den Einbau radioaktiv markierter Ribonucleosid-5'-triphosphatvorläufer in die rekombinanten RNA-Produkte erfolgt.

35. Verfahren nach Anspruch 25 oder 26, wobei das Nachweisen durch den Einbau chemisch modifizierter Ribonucleosid-5'-triphosphatvorläufer in die rekombinanten RNA-Produkte erfolgt.

36. Verfahren nach Anspruch 35, wobei die chemisch modifizierten Ribonucleosid-5'-triphosphatvorläufer biotinyliert sind.

37. Verfahren nach Anspruch 35, wobei die chemisch modifizierten Ribonucleosid-5'-triphosphatvorläufer eine Fluoreszenz zeigen.

38. Verfahren nach Anspruch 25 oder 26, wobei der Nachweis durch Binden RNA-spezifischer chromogener oder fluorogener Farbstoffe an die rekombinanten RNA-Produkte erfolgt.

39. Verfahren nach Anspruch 25 oder 26, wobei der Nachweis durch physikalische Verfahren erfolgt.

40. Verfahren nach Anspruch 25 oder 26, wobei in Stufe (c) die auf RNA gerichtete RNA-Polymerase Qβ-Replikase ist.

41. Verfahren nach Anspruch 25 oder 25, wobei die Inkubationszeit in Stufe (c) ausreichend kurz ist, so daß die Zahl der rekombinanten RNA-Produktstränge die Zahl der Polymerasemoleküle nicht übersteigt, was dazu führt, daß die Zahl der rekombinanten RNA-Produktmoleküle linear proportional zur Zahl der ursprünglich hybridisierten rekombinanten RNA-Sondenmoleküle ist.

42. Verfahren nach Anspruch 25 oder 26, wobei die Inkubationszeit in Stufe (c) ausreichend lang ist, so daß die Zahl der rekombinanten RNA-Produktstränge die Zahl der Polymerasemoleküle übersteigt, was dazu führt, daß die Zahl der rekombinanten RNA-Produktmoleküle proportional zum Logarithmus der Zahl der ursprünglich hybridisierten rekombinanten RNA-Sondenmoleküle ist.

## Revendications

1. Molécule sonde d'ARN recombinant à simple brin, réplicable et hybridable, comprenant:
(a) une séquence de reconnaissance pour la fixation d'une ARN polymérase dirigée sur l'ARN;
(b) une séquence requise pour l'initiation de la synthèse d'un brin de produit par la polymérase; et
(c) une séquence d'ARN hétérologue complémentaire d'une séquence d'acide nucléique spécifique d'un agent infectieux et insérée en un site spécifique dans la région interne de la molécule recombinante, de telle sorte que la séquence hétérologue de la molécule sonde puisse avoir une conformation à simple brin pour l'hybridation avec une séquence cible et puisse former des structures secondaires pour une réplication efficace.

2. Molécule sonde d'ARN recombinant selon la revendication 1, dans laquelle la séquence de reconnaissance pour la liaison d'une ARN polymérase dirigée sur l'ARN est dans une région interne de la molécule.

3. Molécule sonde d'ARN recombinant selon la revendication 1, dans laquelle la séquence requise pour l'initiation de la synthèse du brin de produit est une séquence 3'-terminale riche en cytidine.

4. Molécule sonde d'ARN recombinant selon la revendication 1, dans laquelle l'ARN polymérase dirigée contre l'ARN est de la Qβ réplicase.

5. Molécule sonde d'ARN recombinant selon la revendication 1, dans laquelle la molécule est une matrice d'ARN variant pour Qβ réplicase ou un de ses mutants.

6. Molécule sonde d'ARN recombinant selon la revendication 5, dans laquelle la matrice d'ARN variant est de l'ARN de MDV-1 ou un de ses mutants.

7. Molécule sonde d'ARN recombinant selon la revendication 6, dans laquelle l'ARN de MDV-1 est de l'ARN de MDV-1 (+).

8. Molécule sonde d'ARN recombinant selon la revendication 6, dans laquelle l'ARN de MDV-1 est de l'ARN de MDV-1 (-).

9. Molécule sonde d'ARN recombinant selon la revendication 7, dans laquelle la séquence hétérologue intéressante est insérée entre les nucléotides 63 et 64.

10. Molécule sonde d'ARN recombinant selon la revendication 1, dans laquelle la molécule est un produit de transcription dérivé d'un plasmide recombinant par incubation avec une ARN polymérase dirigée sur de l'ADN.

11. Molécule sonde d'ARN recombinant selon la revendication 10, dans laquelle la molécule est une matrice d'ARN variant pour Qβ réplicase ou un de ses mutants.

12. Molécule sonde d'ARN recombinant selon la revendication 11, dans laquelle la matrice d'ARN variant est de l'ARN de MDV-1 ou un de ses mutants.

13. Molécule sonde d'ARN recombinant selon la revendication 12, dans laquelle l'ARN de MDV-1 est de l'ARN de MDV-1 (+).

14. Molécule sonde d'ARN recombinant selon la revendication 12, dans laquelle l'ARN de MDV-1 est de L'ARN de MDV-1 (-).

15. Molécule sonde d'ARN recombinant selon la revendication 13, dans laquelle la séquence hétérologue est insérée entre les nucléotides 63 et 64.

16. Molécule sonde d'ARN recombinant selon la revendication 1, dans laquelle l'agent infectieux est un virus.

17. Molécule sonde d'ARN recombinant selon la revendication 1, dans laquelle l'agent infectieux est un viroïde ou un virusoïde.

18. Molécule sonde d'ARN recombinant selon la revendication 1, dans laquelle l'agent infectieux est un procaryote.

19. Molécule sonde d'ARN recombinant selon la revendication 18, dans laquelle le procaryote est une bactérie.

20. Molécule sonde d'ARN recombinant selon la revendication 1, dans laquelle l'agent infectieux est un eucaryote.

21. Molécule sonde d'ARN recombinant selon la revendication 20, dans laquelle l'eucaryote est un protozoaire parasite.

22. Molécule sonde d'ARN recombinant selon la revendication 21, dans laquelle le protozoaire parasite provoque le paludisme.

23. Molécule sonde d'ARN recombinant à simple brin, réplicable et hybridable, comprenant:
(a) une séquence de reconnaissance pour la liaison d'une ARN polymérase dirigée sur l'ARN;
(b) une séquence requise pour l'initiation de la synthèse d'un brin de produit par la polymérase; et
(c) une séquence d'ARN hétérologue autre que la séquence 5'-AGUCAGGCACCGUGUAUGAAAUCUAACAAU-3', complémentaire d'une séquence génique spécifique ou d'une portion de celle-ci insérée en un site spécifique dans la région interne de la molécule recombinante, de telle sorte que la séquence hétérologue de la molécule sonde puisse avoir une conformation à simple brin pour l'hybridation avec une séquence cible et puisse former des structures secondaires pour une réplication efficace.

24. Molécule sonde d'ARN recombinant selon la revendication 23, dans laquelle la séquence génique spécifique est un allèle ou une portion de celui-ci.

25. Procédé pour la détermination de la présence ou de la concentration d'une séquence spécifique d'acides nucléiques d'un agent infectieux dans un échantillon, comprenant les étapes de:
(a) formation d'un complexe spécifique entre la molécule sonde d'ARN recombinant selon la revendication 1 et la séquence spécifique d'acides nucléiques d'un agent infectieux, par incubation de l'échantillon avec les molécules sondes d'ARN recombinant dans des conditions appropriées et pendant une durée suffisante pour permettre aux séquences nucléotidiques complémentaires de s'hybrider;
(b) élimination des molécules sondes d'ARN recombinant non hybridées du mélange réactionnel;
(c) incubation du mélange réactionnel avec une ARN polymérase dirigée sur l'ARN et capable de synthétiser des copies additionnelles des molécules sondes d'ARN recombinant qui sont hybridées en la séquence spécifique d'acides nucléiques de l'agent infectieux; et
(d) détection des molécules sondes d'ARN recombinant synthétisées dans l'étape (c), ce qui permet de déterminer la présence ou la concentration de la séquence spécifique d'acides nucléiques de l'agent infectieux.

26. Procédé pour la détermination de la présence ou de la concentration d'une séquence génique spécifique ou d'une portion de celle-ci dans un échantillon, comprenant les étapes de:
(a) formation d'un complexe spécifique entre la molécule sonde d'ARN recombinant selon la revendication 23 et la séquence génique spécifique ou une portion de celle-ci, par incubation de l'échantillon avec les molécules sondes d'ARN recombinant dans des conditions appropriées et pendant une durée suffisante pour permettre aux séquences nucléotidiques complémentaires de s'hybrider;
(b) élimination des molécules sondes d'ARN recombinant non hybridées du mélange réactionnel;
(c) incubation du mélange réactionnel avec une ARN polymérase dirigée sur l'ARN et capable de synthétiser des copies additionnelles des molécules sondes d'ARN recombinant qui sont hybridées en la séquence génique spécifique ou une portion de celle-ci; et
(d) détection des molécules sondes d'ARN recombinant synthétisées dans l'étape (c), ce qui permet de déterminer la présence ou la concentration de la séquence génique spécifique ou d'une portion de celle-ci.

27. Procédé selon la revendication 25, dans lequel la séquence spécifique d'acides nucléiques de l'agent infectieux dans l'échantillon est fixée sur un support solide.

28. Procédé selon la revendication 26, dans lequel la séquence génique spécifique ou une portion de celle-ci dans l'échantillon est fixée sur un support solide.

29. Procédé selon l'une des revendications 27 ou 28, dans lequel le support solide est une nitrocellulose ou une membrane de nylon.

30. Procédé selon la revendication 25, dans lequel dans l'étape (a) la séquence spécifique d'acides nucléiques de l'agent infectieux et la molécule sonde d'ARN recombinant sont en solution.

31. Procédé selon la revendication 26, dans lequel dans l'étape (a) la séquence génique spécifique ou une portion de celle-ci et la molécule sonde d'ARN recombinant sont en solution.

32. Procédé selon la revendication 30, dans lequel dans l'étape (b) les molécules sondes d'ARN recombinant hybridées sont séparées des molécules sondes non hybridées par la capture de la séquence spécifique d'acides nucléiques de l'agent infectieux sur un support solide.

33. Procédé selon la revendication 31, dans lequel dans l'étape (b) les molécules sondes d'ARN recombinant hybridées sont séparées des molécules sondes non hybridées par la capture de la séquence génique spécifique ou d'une portion de celle-ci sur un support solide.

34. Procédé selon l'une des revendications 25 ou 26, dans lequel la détection est effectuée par l'incorporation de précurseurs de ribonucléoside 5'-triphosphate à marquage radioactif dans les produits d'ARN recombinant.

35. Procédé selon l'une des revendications 25 ou 26, dans lequel la détection est effectuée par l'incorporation de précurseurs de ribonucléoside 5'-triphosphate modifiés chimiquement dans les produits d'ARN recombinant.

36. Procédé selon la revendication 35, dans lequel les précurseurs de ribonucléoside 5'-triphosphate modifiés chimiquement sont biotinylés.

37. Procédé selon la revendication 35, dans lequel les précurseurs de ribonucléoside 5'-triphosphate modifiés chimiquement sont fluorescents.

38. Procédé selon l'une des revendications 25 ou 26, dans lequel la détection est effectuée par la fixation de colorants chromogènes ou fluorogènes spécifiques de l'ARN aux produits d'ARN recombinants.

39. Procédé selon l'une des revendications 25 ou 26, dans lequel la détection est effectuée par des méthodes physiques.

40. Procédé selon l'une des revendications 25 ou 26, dans lequel dans l'étape (c) l'ARN polymérase dirigée sur de l'ARN est de la Qβ réplicase.

41. Procédé selon l'une des revendications 25 ou 26, dans lequel la durée d'incubation dans l'étape (c) est suffisamment courte pour que le nombre de brins de produit d'ARN recombinant ne dépasse pas le nombre de molécules de polymérase, avec pour résultat que le nombre de molécules de produit d'ARN recombinant est linéairement proportionnel au nombre de molécules sondes d'ARN recombinant hybridées à l'origine.

42. Procédé selon l'une des revendications 25 ou 26, dans lequel la durée d'incubation dans l'étape (c) est suffisamment longue pour que le nombre de brins de produit d'ARN recombinant dépasse le nombre de molécules de polymérase, avec pour résultat que le nombre de molécules de produit d'ARN recombinant est proportionnel au logarithme du nombre de molécules sondes d'ARN recombinant hybridées à l'origine.
